# EUROPEAN PATENT SPECIFICATION

(11) **EP 1 809 308 B1**
(45) Date of publication and mention of the grant of the patent: **18.05.2011**
(21) Application number: 04822414.1
(22) Date of filing: 08.11.2004
(51) Int. Cl.: A61K 35/00, A01N 25/00, A01N 25/08, A01N 25/24

(54) **AN ADJUVANT COMPOSITION FOR USE WITH HERBICIDES, PESTICIDES, INSECTICIDES, OVICIDES AND FUNGICIDES AND METHOD OF APPLICATION**
ADJUVANT ZUSAMMENSETZUNG ZUR VERWENDUNG MIT HERBIZIDEN, PESTIZIDEN, INSEKTIZIDEN, OVIZIDEN UND FUNGIZIDEN UND ANWENDUNGSVERFAHREN
FORMULE D'ADDITIF POUR HERBICIDES, PESTICIDES, INSECTICIDES, OVICIDES ET FONGICIDES ET MÉTHODE D'EMPLOI DE LADITE FORMULE

(43) Date of publication of application: 25.07.2007
(73) Proprietor: Oro-Agri, Inc., Trophy Club TX 76262 (US)
(72) Inventor: PULLEN, Erroll, M., Bantry Bay 8005 (ZA)
(74) Representative: Kador & Partner
(86) International application number: PCT/US2004/035512
(87) International publication number: WO 2006/052228

(56) References cited:
- WO-A-01/26457
- WO-A-97/16975
- WO-A-03/056917
- US-A1- 2003 035 852
- US-A1- 2004 138 176
- US-B1- 6 258 369
- US-B2- 6 582 712

## Description

### Technical Field

An adjuvant for use with systemic herbicides, pesticides, insecticides, ovicides and fungicide on plants, trees, fruits and vegetables.

### Background Art

Various insects such as lice, ticks, mites and aphides attack untreated and unprotected trees and plants. Moreover, fungi left uncontrolled can damage and even destroy plants and trees including crops associated therewith.

In the past, various oils have been used to control insects and mites. Recently, however, renewed attention has focused on the use of oils as a natural substitute for traditional insecticides with attendant toxic and other dangerous side effects.

These oils include horticultural oils that are highly refined petroleum products than can be mixed with water for application for control of target insect and mite pests without deleterious effects. Modern horticultural oils do not include vegetable, fish or whale oils.

Horticultural spray oils are the low toxicity alternative to broad-spectrum insecticides. Since the mechanism of insect and mite control with spray oils is by suffocation and/or repellency of egg laying females, there is no requirement for the addition of toxic chemicals. These properties are a valuable and well-recognized component of the practice of integrated pest management where oil spraying is intrinsically linked to natural control of pests by predators and parasitoids. Horticultural spray oils are formulated on highly refined clear oil with a minimum of nonionic surfactant. Independent environmental impact studies have shown that D-C-TRON has no detrimental effect on the environment. Mammalian toxicity studies published in the American Journal of Industrial Medicine have shown that oils at this refinement level are non-toxic and non-carcinogenic.

Generally, oil sprays are safe to humans. These oil sprays have little, if any, negative effect on wildlife and non-target insects in the environment. Furthermore, oil sprays are less toxic due to the method by which they kill target pests. In particular, the thin film of oil covers the target insect or mite and plugs the spiracles or pores through which the pests or parasites breathe. The cause of death is primarily suffocation. Large, motile insects and animals that breathe by another method are not affected by these oils.

Another advantage of oil applications is the absence of objectionable odors. In addition, oils are relatively inexpensive and significantly less expensive than many insecticides.

Unfortunately, there are limitations to the use of oil treatments. For example, oils are only effective against those pests that are thoroughly coated by the spray solution. This usually means that only small, immobile or slow moving pests that are exposed on the surface of the plant or tree at the time of application will be controlled.

Since oil sprays only work by contracting and covering the target pest, thorough application is essential. Missed surface areas provide a safe refuge for the target pests.

U.S. 6,258,369 and U.S. 6,277,389 disclose a non-toxic aqueous pesticide for application on plants and animals comprising at least one surfactant and at least one high terpene containing natural oil. The pesticide is used to effectively control insects and parasites such as darkling beetles, lice, ticks, mites, flies, aphides, mosquitoes and chiggers found on plants and animals.

U.S. 5,693,344 shows a hazard-free method for controlling insects using a non-toxic composition in the form of a fragrance and crystalline particles which puncture directly through the exoskeleton of an insect. In operation, the particles work themselves between the insect's protective body plates and then puncture the exoskeleton permitting entry of the fragrance into the body of the insect. Once inside, the particles absorb up to four times their weight of the vital body fluids of the insect and the fragrance has a neural effect on the insect.

U.S. 5,143,939 shows a method of treating soil and agricultural crops for controlling worms and nematodes comprising a nonionic surfactant, namely an alkyloxypolyethyleneoxyethanol used as the sole active ingredient to control fungus, mites, worms, termites, nematodes and other insects.

U.S. 4,379,168 relates to pesticides containing d-limonene as an insect-killing ingredient with surfactants or emulsifiers and water. The pesticide compositions are liquids designed for use as a dip to rid small animals of fleas and ticks, a spray to kill fleas and ticks on small animals and in the kennels of small animals; a spray to kill flies on small animals and in the kennels of small animals; and a spray or liquid to rid household areas of cockroaches and other insect pests.

U.S. 6,248,710 B1 discloses a water-soluble or water-dispersible material for deposition onto a fabric substrate during a treatment process comprising polysaccharide structure having at least one substituent benefit agent group and optionally, one or more other substituent groups. The polysaccharide structure has one or more regions with at least 3, preferably at least 4 consecutive unsubstituted saccharide rings.

WO 03/056917 discloses a composition for use with plants, trees, fruits and vegetables comprising at least one surfactant and at least one high terpene containing oil.

WO 97/16975 concerns a herbicidal composition containing a terpene compound, a terpene derivative or an essential oil comprising a terpene compound or derivative.

### Disclosure of Invention

The present invention relates to a composition according to claim 1.

High terpene containing natural oil as used herein means those natural oils having a terpene content of at least 50 per cent. It is preferable that the high terpene natural oil contains at least 65 per cent. Suitable high terpene containing natural oils includes oil from conifers such as citrus peel oils, preferably orange oil, grapefruit oil, lemon oil or pine oil. Of these, orange oil is preferred and cold pressed orange oil the most preferred. The preferred terpene content is from about 80 per cent to about 90 per cent and most preferred from about 85 per cent to about 87 per cent, all by weight.

The amount of high terpene containing natural oils in the adjuvant composition depends upon the amount of terpenes in the specific oil used. Generally, the adjuvant composition contains from about 2 per cent by weight to about 8 per cent by weight of high terpene containing natural oil, preferably about 5 per cent by weight.

Anionic and nonionic surfactants are acceptable for use in the adjuvant composition of the present invention. Anionic surfactants such as salts of fatty acids, alkyl sulphates, alkyl ether sulphonates and alkyl aryl sulphonates are preferred.

The adjuvant composition may also contain preservatives, pH neutralizers and/or clarifiers or stabilizers. The balance of the adjuvant composition is water.

In use, the adjuvant, when combined with systemic herbicides, pesticides, insecticides, ovicides and fungicides, is diluted and sprayed or misted on plants, trees, fruits or vegetables.

When so applied, the adjuvant composition is effective as an adjuvant in enhancing the effect of systemic herbicides, insecticides, ovicides and fungicides that are applied to control various diseases, pests and insects including darkling beetles, lice, ticks, mites, flies, aphides, thrips, mealybugs, mosquitoes and chiggers.

The adjuvant composition is also effective as an adjuvant in enhancing fungicides in controlling fungi. While not to be bound by theory, absorption of fungicide, once blended with the adjuvant, is increased both in speed as well as percentage absorbed.

Finally, the adjuvant composition as an adjuvant enhances water penetration and absorption by the soil as well as decreases water logging. These better soil conditions lead to improved root and plant growth.

The present invention relates to a composition according to claim 1 being formulated with systemic herbicides, pesticides, insecticides, ovicides and fungicides for use with various trees, plants, fruits and vegetables.

The adjuvant composition comprises at least one surfactant and at least one high terpene containing oil to enhance the effectiveness of pesticides, insecticides, ovicides and fungicides in controlling pests, insects and fungi. Furthermore, the adjuvant composition may be used as an adjuvant with contact pesticides.

High terpene containing natural oil as used herein means those natural oils having a terpene content of at least 50 per cent. It is preferable that the high terpene natural oil contains at least 65 per cent. Suitable high terpene containing natural oils includes oil from conifers such as citrus peel oils, preferably orange oil, grapefruit oil, lemon oil or pine oil. Of these, orange oil is preferred and cold pressed orange oil the most preferred. The preferred terpene content is from 80 per cent to 90 per cent and most preferred from 85 per cent to 87 per cent, all by weight.

The amount of high terpene containing natural oils in the adjuvant composition depends upon the amount of terpenes in the specific oil used. The adjuvant composition contains from 2 per cent by weight to 8 per cent by weight of high terpene containing natural oil, preferably about 5 per cent by weight.

Anionic and nonionic surfactants are acceptable for use in the adjuvant composition of the present invention. Anionic surfactants such as salts of fatty acids, alkyl sulphates, alkyl ether sulphonates and alkyl aryl sulphonates are preferred. Examples of such surfactants may include from 8 per cent to 12 per cent sulfonic acid, preferably about 10 per cent sulfonic acid; from 5 per cent to 9 per cent sodium laurel sulfate, preferably about 6.8 per cent sodium laurel sulfate; from 6 per cent to 10 per cent alcohol ethoxylate, preferably about 8.2 per cent alcohol ethoxylate; and from 1 per cent to 3 per cent olefin sulfonate, preferably about 1.7 olefin sulfonate, all by weight.

The adjuvant composition contains from 20 per cent to 34 per cent surfactant(s), preferably from 25 per cent to 30 per cent surfactant(s) and most preferably about 26.7 per cent surfactant(s), all by weight.

The adjuvant composition may also include butylated hydroxytoluene, p-Hydroxybenzoic acid and/or sodium tetraborate decahydrate. The range of butylated hydroxytoluene is from 0.05 per cent to 0.15 per cent and preferably about 0.10 per cent, all by weight. The range of sodium tetraborate decahydrate is from 0.89 per cent to 1.09 per cent and preferably about 0.99 per cent, all by weight. The range of p- Hydroxybenzoic acid is from 0.25 per cent to 0.35 per cent and preferably about 0.30 per cent, all by weight. Generally, the adjuvant composition contains from 1.39 per cent to 1.89 per cent preservative(s), preferably about 1.64 per cent preservative(s), all by weight.

In addition, a bactericide is from 0.05 per cent to 0.15 per cent and preferably about 0.10 per cent, all by weight may be added.

Caustic crystals such as sodium hydroxide may be added in an amount of from 1.25 per cent to 1.37 per cent by weight to neutralize the adjuvant composition to a pH of from 7.75 to 9.

A clarifier or stabilizer such as urea may be added in an amount of from 0.59 per cent to 0.99 per cent and preferably about 0.79 per cent, all by weight.

The balance of the adjuvant composition is made up by water.

The preferred adjuvant composition comprises about 5 per cent cold pressed orange oil, about 6.8 per cent sodium lauryl sulfate, about 8.2 per cent of alcohol ethoxylate, about 1.7 per cent sodium olefin sulfonate, about 10 per cent dodecylbenzene sulphonic acid, about 0.1 per cent antioxidant such as butylate hydroxytoluene, about 0.30 per cent preservative such as p-Hydroxybenzoic acid, about 0.1 per cent bactericide, about 0.99 per cent fungicide such as sodium tetraborate decahydrate, about 0.79 per cent clarifier such as urea and about 1.31 per cent neutralizer such as sodium hydroxide with the balance a diluent such as water, all by weight.

In use, the adjuvant composition is combined with a herbicide, pesticide, insecticide, ovicide or fungicide effective as either a contact or systemic herbicide, pesticide, insecticide, ovicide or fungicide. An effective range for the composition is from 1/2 part to 8 parts adjuvant to 1000 parts herbicide, pesticide, insecticide, ovicide or fungicide and water. The preferred range for the composition is from 11/2 parts to 4 parts adjuvant composition to 1000 parts herbicide, pesticide, insecticide, ovicide, fungicide. The preferred concentration of adjuvant concentration is about 2 parts adjuvant composition to 1000 parts herbicide, pesticide, insecticide, ovicide or fungicide.

The combined adjuvant composition and herbicide, pesticide, insecticide, ovicide or fungicide is applied to plants or row crops such as most vegetables at an application rate of about five (5) liters or less per 4047 m² (per acre).

The combined adjuvant composition and herbicide, pesticide, insecticide, ovicide or fungicide is applied to trees or orchards at an application rate of about eight (8) liters or less per 4047 m² (per acre).

### Best Mode for Carrying Out the Invention

The present invention relates to an adjuvant for use with systemic herbicides, pesticides, insecticides, ovicides and fungicides formulated for use with various trees, plants, fruits and vegetables. The composition comprises at least one surfactant and at least one high terpene containing oil to enhance the effectiveness of pesticides, insecticides, ovicides and fungicides in controlling pests, insects and fungi. The invention also includes the method of application of the composition. Furthermore, the composition may be used as an adjuvant with contact pesticides.

High terpene containing natural oil as used herein means those natural oils having a terpene content of at least about 50 per cent. It is preferable that the high terpene natural oil contains at least about 65 per cent. Suitable high terpene containing natural oils includes oil from conifers such as citrus peel oils, preferably orange oil, grapefruit oil, lemon oil or pine oil. Of these, orange oil is preferred and cold pressed orange oil the most preferred. The preferred terpene content is from about 80 per cent to about 90 per cent and most preferred from about 85 per cent to about 87 per cent, all by weight.

The amount of high terpene containing natural oils in the composition depends upon the amount of terpenes in the specific oil used. Generally, the composition contains from about 2 per cent by weight to about 8 per cent by weight of high terpene containing natural oil, preferably about 5 per cent by weight.

Anionic and nonionic surfactants are acceptable for use in the composition of the present invention. Anionic surfactants such as salts of fatty acids, alkyl sulphates, alkyl ether sulphonates and alkyl aryl sulphonates are preferred. Examples of such surfactants may include from about 8 per cent to about 12 per cent sulfonic acid, preferably about 10 per cent sulfonic acid; from about 5 per cent to about 9 per cent sodium laurel sulfate, preferably about 6.8 per cent sodium laurel sulfate; from about 6 per cent to about 10 per cent alcohol ethoxylate, preferably about 8.2 per cent alcohol ethoxylate; and from about 1 per cent to about 3 per cent olefin sulfonate, preferably about 1.7 olefin sulfonate, all by weight.

Generally, the composition contains from about 20 per cent to about 34 per cent surfactant(s), preferably from about 25 per cent to about 30 per cent surfactant(s) and most preferably about 26.7 per cent surfactant(s), all by weight.

The composition may also include butylated hydroxytoluene, p-Hydroxybenzoic acid and/or sodium tetraborate decahydrate. The range of butylated hydroxytoluene is from about 0.05 per cent to about 0.15 per cent and preferably about 0.10 per cent, all by weight. The range of sodium tetraborate decahydrate is from about 0.89 per cent to about 1.09 per cent and preferably about 0.99 per cent, all by weight. The range of p-Hydroxybenzoic acid is from about 0.25 per cent to about 0.35 per cent and preferably about 0.30 per cent, all by weight. Generally, the composition contains from about 1.39 per cent to about 1.89 per cent preservative(s), preferably about 1.64 per cent preservative(s), all by weight.

In addition, a bactericide is from about 0.05 per cent to about 0.15 per cent and preferably about 0.10 per cent, all by weight may be added.

Caustic crystals such as sodium hydroxide may be added in an amount of from about 1.25 per cent to about 1.37 per cent by weight to neutralize the composition to a pH of from about 7.75 to about 9.

A clarifier or stabilizer such as urea may be added in an amount of from about 0.59 per cent to about 0.99 per cent and preferably about 0.79 per cent, all by weight.

The balance of the composition is made up by water.

The preferred composition comprises about 5 per cent cold pressed orange oil, about 6.8 per cent sodium lauryl sulfate, about 8.2 per cent of alcohol ethoxylate, about 1.7 per cent sodium olefin sulfonate, about 10 per cent dodecylbenzene sulphonic acid, about 0.1 per cent antioxidant such as butylate hydroxytoluene, about 0.30 per cent preservative such as p-Hydroxybenzoic acid, about 0.1 per cent bactericide, about 0.99 per cent fungicide such as sodium tetraborate decahydrate, about 0.79 per cent clarifier such as urea and about 1.31 per cent neutralizer such as sodium hydroxide with the balance a diluent such as water, all by weight.

In use, the adjuvant composition is combined with a herbicide, pesticide, insecticide, ovicide or fungicide effective as either a contact or systemic herbicide, pesticide, insecticide, ovicide or fungicide. An effective range for the adjuvant composition is from about 1/2 part to about 8 parts adjuvant to 1000 parts herbicide, pesticide, insecticide, ovicide or fungicide and water. The preferred range for the adjuvant composition is from about 1 1/2 parts to about 4 parts adjuvant composition to 1000 parts herbicide, pesticide, insecticide, ovicide, fungicide. The preferred concentration of adjuvant concentration is about 2 parts adjuvant composition to 1000 parts herbicide, pesticide, insecticide, ovicide or fungicide.

The combined adjuvant composition and herbicide, pesticide, insecticide, ovicide or fungicide is applied to plants or row crops such as most vegetables at an application rate of about five (5) liters or less per acre.

The combined adjuvant composition and herbicide, pesticide, insecticide, ovicide or fungicide is applied to trees or orchards at an application rate of about eight (8) liters or less per acre.

## Claims

1. A composition obtainable by combining an adjuvant composition with an herbicide, a pesticide, an insecticide, an ovicide or a fungicide
at a ratio of from 1/2 part to 8 parts adjuvant composition to 1000 parts of said herbicide, said pesticide, said insecticide, said ovicide, or said fungicide;
wherein the adjuvant composition comprises
from 20 per cent to 34 per cent of at least one surfactant, wherein the at least one surfactant is selected from the group of anionic and nonionic surfactants, and
from 2 per cent to 8 per cent terpene-containing natural oil, said terpene-containing natural oil having a terpene content of at least 50 per cent, all by weight.

2. The composition of claim 1, obtainable by combining an adjuvant composition with an herbicide, a pesticide, an insecticide, an ovicide or a fungicide at a ratio of from 1/2 part to 8 parts adjuvant composition to 1000 parts of said herbicide, said pesticide, said insecticide, said ovicide, or said fungicide and water.

3. The composition of claim 1 or 2, wherein said adjuvant composition comprises from 25 per cent to 30 per cent by weight of the least one surfactant.

4. The composition of claim 1 or 2, wherein said adjuvant composition comprises about 27 per cent by weight of the least one surfactant and about 5 per cent by weight of said terpene containing natural oil.

5. The composition of claim 1 or 2, wherein said at least one surfactant comprises sulfonic acid, sodium laurel sulfate, alcohol ethoxylate and olefin sulfonate.

6. The composition of claim 1 or 2, wherein said at least one surfactant comprises from 8 per cent to 12 per cent sulfonic acid; from 5 per cent to 9 per cent sodium laurel sulfate; from 6 per cent to 10 per cent alcohol ethoxylate and from 1 per cent to 3 per cent olefin sulfonate, all by weight.

7. The composition of claim 1 or 2, wherein said at least one surfactant comprises about 10 per cent cent sulfonic acid; about 6.8 per cent sodium laurel sulfate; about 8.2 per cent alcohol ethoxylate and about 1.7 per cent olefin sulfonate, all by weight.

8. The composition of claim 1 or 2, wherein said adjuvant composition further comprises sodium tetraborate decahydrate.

9. The composition of claim 1 or 2, wherein said adjuvant composition further comprises 0.89 per cent to 1.09 per cent by weight sodium tetraborate decahydrate.

10. The composition of claim 1 or 2, wherein said adjuvant composition further comprises about 0.99 per cent by weight sodium tetraborate decahydrate.

11. The composition of claim 1 or 2, wherein said adjuvant composition has a pH of 7.75 to 9.

12. The composition of claim 1 or 2, wherein said adjuvant composition further comprises from 1.25 to 1.37 per cent by weight sodium hydroxide.

13. The composition of claim 1 or 2, wherein said adjuvant composition comprises about 5 per cent cold pressed orange oil, about 6.8 per cent sodium lauryl sulfate, about 8.2 per cent alcohol ethoxylate, about 1.7 per cent olefin sulfonate, about 10 per cent dodecylbenzene sulphonic acid and about 0.99 per cent sodium tetraborate decahydrate with the balance water, all by weight.

14. The composition of claim 1 or 2, wherein said adjuvant composition further comprises from 0.55 per cent to 0.99 per cent by weight urea.

15. The composition of claim 1 or 2, wherein said adjuvant composition further comprises from 0.05 to 0.15 per cent by weight butylated hydroxytoluene.

16. The composition of claim 1 or 2, wherein said adjuvant composition further comprises about 0.10 per cent by weight butylated hydroxytoluene.

17. The composition of claim 1 or 2, obtainable by combining an adjuvant composition with an herbicide, a pesticide, an insecticide, an ovicide or a fungicide at a ratio of from 1/2 part to 4 parts adjuvant composition to 1000 parts of said herbicide, said pesticide, said insecticide, said ovicide, or said fungicide.

18. The composition of claim 1 or 2, obtainable by combining an adjuvant composition with an herbicide, a pesticide, an insecticide, an ovicide or a fungicide at a ratio of about 2 parts adjuvant composition to 1000 parts of said herbicide, said pesticide, said insecticide, said ovicide, or said fungicide.

19. A method of controlling pests, insects or fungi by applying a composition according to any of the preceding claims.

20. The method of claim 19, wherein said composition is applied at a rate of about 5 liters per 4047 m² (per acre) on plants and row crops.

21. The method of claim 19, wherein said composition is applied at a rate of about 8 liters per 4047 m² (per acre) on trees and orchards.

22. The method of claim 19, wherein said composition is sprayed or misted on plants, trees, fruits or vegetables.

## Patentansprüche

1. Zusammensetzung, die durch Kombinieren einer Adjuvanszusammensetzung mit einem Herbizid, einem Pestizid, einem Insektizid, einem Ovizid oder einem Fungizid
in einem Verhältnis von 1/2 bis 8 Teile der Adjuvanszusammensetzung auf 1000 Teile des Herbizids, des Pestizids, des Insektizids, des Ovizids oder des Fungizids erhalten werden kann;
wobei die Adjuvanszusammensetzung folgendes aufweist:
20 bis 34 % von zumindest einem oberflächenaktiven Mittel, wobei das zumindest eine oberflächenaktive Mittel aus der Gruppe von anionischen und nichtionischen oberflächenaktiven Mitteln ausgewählt ist, und
2 bis 8 % Terpen enthaltendes natürliches Öl, wobei das Terpen enthaltende natürliche Öl einen Terpengehalt von mindestens 50 % hat, und zwar jeweils auf das Gewicht bezogen.

2. Zusammensetzung nach Anspruch 1, die durch Kombinieren einer Adjuvanszusammensetzung mit einem Herbizid, einem Pestizid, einem Insektizid, einem Ovizid oder einem Fungizid in einem Verhältnis von 1/2 bis 8 Teile der Adjuvanszusammensetzung auf 1000 Teile des Herbizids, des Pestizids, des Insektizids, des Ovizids oder des Fungizids und Wasser erhalten werden kann.

3. Zusammensetzung nach Anspruch 1 oder 2, wobei die Adjuvanszusammensetzung 25 bis 30 Gew.-% des zumindest einen oberflächenaktiven Mittels umfaßt.

4. Zusammensetzung nach Anspruch 1 oder 2, wobei die Adjuvanszusammensetzung etwa 27 Gew.-% des zumindest einen oberflächenaktiven Mittels und etwa 5 Gew.-% des Terpen enthaltenden natürlichen Öls mfaßt.

5. Zusammensetzung nach Anspruch 1 oder 2, wobei das zumindest eine oberflächenaktive Mittel Sulfonsäure, Natriumlaurelsulfat, Alkoholethoxylat und Olefinsulfonat umfaßt.

6. Zusammensetzung nach Anspruch 1 oder 2, wobei das zumindest eine oberflächenaktive Mittel 8 bis 12 % Sulfonsäure, 5 bis 9 % Natriumlaurelsulfat, 6 bis 10 % Alkoholethoxylat und 1 bis 3 % Olefinsulfonat umfaßt, und zwar jeweils auf das Gewicht bezogen.

7. Zusammensetzung nach Anspruch 1 oder 2, wobei das zumindest eine oberflächenaktive Mittel etwa 10 % Sulfonsäure, etwa 6,8 % Natriumlaurelsulfat, etwa 8,2 % Alkoholethoxylat und etwa 1,7 % Olefinsulfonat umfaßt, und zwar jeweils auf das Gewicht bezogen.

8. Zusammensetzung nach Anspruch 1 oder 2, wobei die Adjuvanszusammensetzung ferner Natriumtetraboratdecahydrat umfaßt.

9. Zusammensetzung nach Anspruch 1 oder 2, wobei die Adjuvanszusammensetzung ferner 0,89 bis 1,09 Gew.-% Natriumtetraboratdecahydrat umfaßt.

10. Zusammensetzung nach Anspruch 1 oder 2, wobei die Adjuvanszusammensetzung ferner etwa 0,99 Gew.-% Natriumtetraboratdecahydrat umfaßt.

11. Zusammensetzung nach Anspruch 1 oder 2, wobei die Adjuvanszusammensetzung einen pH-Wert von 7,75 bis 9 aufweist.

12. Zusammensetzung nach Anspruch 1 oder 2, wobei die Adjuvanszusammensetzung ferner 1,25 bis 1,37 Gew.-% Natriumhydroxid umfaßt.

13. Zusammensetzung nach Anspruch 1 oder 2, wobei die Adjuvanszusammensetzung etwa 5 % kaltgepreßtes Orangenöl, etwa 6,8 % Natriumlaurylsulfat, etwa 8,2 % Alkoholethoxylat, etwa 1,7 % Olefinsulfonat, etwa 10 % Dodecylbenzolsulfonsäure und etwa 0,99 % Natriumtetraboratdecahydrat umfaßt, wobei der Rest Wasser ist, und zwar jeweils auf das Gewicht bezogen.

14. Zusammensetzung nach Anspruch 1 oder 2, wobei die Adjuvanszusammensetzung ferner 0,55 bis 0,99 Gew.-% Harnstoff umfaßt.

15. Zusammensetzung nach Anspruch 1 oder 2, wobei die Adjuvanszusammensetzung ferner 0,05 bis 0,15 Gew.-% butyliertes Hydroxytoluol umfaßt.

16. Zusammensetzung nach Anspruch 1 oder 2, wobei die Adjuvanszusammensetzung ferner etwa 0,10 Gew.-% butyliertes Hydroxytoluol umfaßt.

17. Zusammensetzung nach Anspruch 1 oder 2, die durch Kombinieren einer Adjuvanszusammensetzung mit einem Herbizid, einem Pestizid, einem Insektizid, einem Ovizid oder einem Fungizid in einem Verhältnis von 1/2 bis 4 Teilen der Adjuvanszusammensetzung auf 1000 Teile des Herbizids, des Pestizids, des Insektizids, des Ovizids oder des Fungizids erhalten werden kann.

18. Zusammensetzung nach Anspruch 1 oder 2, die durch Kombinieren einer Adjuvanszusammensetzung mit einem Herbizid, einem Pestizid, einem Insektizid, einem Ovizid oder einem Fungizid in einem Verhältnis von etwa 2 Teilen der Adjuvanszusammensetzung auf 1000 Teile des des Herbizids, des Pestizids, des Insektizids, des Ovizids oder des Fungizids erhalten werden kann.

19. Verfahren zum Kontrollieren von Schädlingen, Insekten oder Pilzen durch Anwenden einer Zusammensetzung nach einem der vorstehenden Ansprüche.

20. Verfahren nach Anspruch 19, wobei die Zusammensetzung in einer Menge von etwa 5 1 pro 4047 m² (pro Acre) auf Pflanzen und Getreide in Reihen aufgebracht wird.

21. Verfahren nach Anspruch 19, wobei die Zusammensetzung in einer Menge von etwa 8 1 pro 4047 m² (pro Acre) auf Bäume und Obstplantagen aufgebracht wird.

22. Verfahren nach Anspruch 19, wobei die Zusammensetzung auf Pflanzen, Bäume, Früchte oder Gemüse gesprüht oder vernebelt wird.

## Revendications

1. Composition pouvant être obtenue par combinaison d'une composition d'adjuvant avec un herbicide, un pesticide, un insecticide, un ovicide ou un fongicide,
en un rapport de 1/2 partie à 8 parties de composition d'adjuvant pour 1000 parties dudit herbicide, dudit pesticide, dudit insecticide, dudit ovicide, ou dudit fongicide ;
dans laquelle la composition d'adjuvant comprend
de 20 % a 34 % d'au moins un tensioactif, l'au moins tensioactif étant choisi dans le groupe des tensioactifs anioniques et non-ioniques, et
de 2 % à 8 % d'huile naturelle terpénique, ladite huile naturelle terpénique ayant une teneur en terpène d'au moins 50 %, tous les pourcentages étant en poids.

2. Composition selon la revendication 1, pouvant être obtenue par combinaison d'une composition d'adjuvant avec un herbicide, un pesticide, un insecticide, un ovicide ou un fongicide en un rapport de 1/2 partie à 8 parties de composition d'adjuvant pour 1000 parties dudit herbicide, dudit pesticide, dudit insecticide, dudit ovicide, ou dudit fongicide, et de l'eau.

3. Composition selon la revendication 1 ou 2, dans laquelle ladite composition d'adjuvant comprend de 25 % à 30 % en poids d'au moins un tensioactif.

4. Composition selon la revendication 1 ou 2, dans laquelle ladite composition d'adjuvant comprend environ 27 % en poids de- l'au moins- tensioactif et environ 5 % en poids de ladite huile naturelle terpénique.

5. Composition selon la revendication 1 ou 2, dans laquelle ledit au moins un tensioactif comprend de l'acide sulfonique, du laurylsulfate de sodium, un alcool éthoxylé et un oléfmesulfonate.

6. Composition selon la revendication 1 ou 2, dans laquelle ledit au moins tensioactif comprend de 8 % à 12 % d'acide sulfonique ; de 5 % à 9 % de laurylsulfate de sodium ; de 6 % à 10 % d'un alcool éthoxylé et de 1 % à 3 % d'un oléfinesulfonate, tous les pourcentages étant en poids.

7. Composition selon la revendication 1 ou 2, dans laquelle ledit au moins un tensioactif comprend environ 10 % d'acide sulfonique ; environ 6,8 % de laurylsulfate de sodium ; environ 8,2 % d'un alcool éthoxylé et environ 1,7 % d'un oléfinesulfonate, tous les pourcentages étant en poids.

8. Composition selon la revendication 1 ou 2, dans laquelle ladite composition d'adjuvant comprend en outre du tétraborate de sodium décahydraté.

9. Composition selon la revendication 1 ou 2, dans laquelle ladite composition d'adjuvant comprend en outre de 0,89 % à 1,09 % en poids de tétraborate de sodium décahydraté.

10. Composition selon la revendication 1 ou 2, dans laquelle ladite composition d'adjuvant comprend en outre environ 0,99 % en poids de tétraborate de sodium décahydraté.

11. Composition selon la revendication 1 ou 2, dans laquelle ladite composition d'adjuvant a un pH de 7,75 à 9.

12. Composition selon la revendication 1 ou 2, dans laquelle ladite composition d'adjuvant comprend en outre de 1,25 à 1,37 % en poids d'hydroxyde de sodium.

13. Composition selon la revendication 1 ou 2, dans laquelle ladite composition d'adjuvant comprend environ 5 % d'huile d'orange pressée à froid, environ 6,8 % de laurylsulfate de sodium, environ 8,2 % d'un alcool éthoxylé, environ 1,7 % d'un oléfinesulfonate, environ 10 % d'acide dodécylbenzènesulfonique et environ 0,99 % de tétraborate de sodium décahydraté, le reste étant de l'eau, tous les pourcentages étant en poids.

14. Composition selon la revendication 1 ou 2, dans laquelle ladite composition d'adjuvant comprend en outre de 0,55 % à 0,99 % en poids d'urée.

15. Composition selon la revendication 1 ou 2, dans laquelle ladite composition d'adjuvant comprend en outre de 0,05 à 0,15 % en poids d'hydroxytoluène butylé.

16. Composition selon la revendication 1 ou 2, dans laquelle ladite composition d'adjuvant comprend en outre environ 0,10 % en poids d'hydroxytoluène butylé.

17. Composition selon la revendication 1 ou 2, pouvant être obtenue par combinaison d'une composition d'adjuvant avec un herbicide, un pesticide, un insecticide, un ovicide ou un fongicide en un rapport de 1/2 partie à 4 parties de composition d'adjuvant pour 1000 parties dudit herbicide, dudit pesticide, dudit insecticide, dudit ovicide, ou dudit fongicide.

18. Composition selon la revendication 1 ou 2, pouvant être obtenue par combinaison d'une composition d'adjuvant avec un herbicide, un pesticide, un insecticide, un ovicide ou un fongicide en un rapport d'environ 2 parties de composition d'adjuvant pour 1000 parties dudit herbicide, dudit pesticide, dudit insecticide, dudit ovicide, ou dudit fongicide.

19. Procédé pour lutter contre les nuisibles, les insectes ou les champignons, par application d'une composition selon l'une quelconque des revendications précédentes.

20. Procédé selon la revendication 19, dans lequel ladite composition est appliquée à un taux d'environ 5 litres pour 4047 m² (par acre) sur des plantes et des cultures en lignes.

21. Procédé selon la revendication 19, dans lequel ladite composition est appliquée à un taux d'environ 8 litres pour 4047 m² (par acre) sur des arbres et des vergers.

22. Procédé selon la revendication 19, dans lequel ladite composition est pulvérisée ou vaporisée sur des plantes, des arbres, des fruits ou des légumes.
